# EUROPÄISCHE PATENTSCHRIFT

(11) **EP 1 049 492 B1**
(45) Veröffentlichungstag und Bekanntmachung des Hinweises auf die Patenterteilung: **22.08.2007**
(21) Anmeldenummer: 99907368.7
(22) Anmeldetag: 14.01.1999
(51) Int. Cl.: A61L 2/00

(54) **Verfahren zur Herstellung von dekontaminierten und sterilisierten Korken**
Process for manufacturing decontaminated and sterilised corks
Procédé de fabrication de bouchons en liège décontaminés et stérilisés

(30) Priorität: 22.01.1998 DE 19802297
(43) Veröffentlichungstag der Anmeldung: 08.11.2000
(73) Patentinhaber: Rudolf Ohlinger GmbH, 67136 Fussgönheim (DE); Francisco Oller, S.A., 17244 Cassa de la Selva (ES); Juvenal Ferreira Da Silva, Lda., 4536 Santa Maria de Lamas (PT)
(72) Erfinder: JÄGER, Jens, D-67466 Lambrecht (DE)
(74) Vertreter: Dipl.-Ing. Heiner Lichti Dipl.-Phys. Dr.rer.nat. Jost Lempert Dipl.-Ing. Hartmut Lasch
(86) Internationale Anmeldenummer: PCT/EP1999/000174
(87) Internationale Veröffentlichungsnummer: WO 1999/037334

(56) Entgegenhaltungen:
- EP-A- 0 360 447
- AU-A- 1 855 583
- ES-A- 2 019 514
- ES-A- 2 020 371
- ES-A- 8 802 283
- FR-A- 2 478 613
- FR-A- 2 645 950
- GB-A- 1 126 233
- IT-B- 1 268 708

## Beschreibung

Die Erfindung betrifft ein Verfahren zur Herstellung von dekontaminierten und sterilisierten Korken.

Naturkorken aus der Baumrinde der Korkeiche (Quercus suber) werden bereits seit dem Altertum in großem Maße zum Verschluß von Flaschen, Rohren usw. verwendet. Flaschenkorken für hochwertige Produkte wie z.B. Wein und Sekt können auf verschiedene Weise hergestellt werden und einen unterschiedlichen Aufbau besitzen. Einerseits ist es möglich, die Korken aus der Korkrinde der Korkeiche auszustanzen, so daß ein einstückiger Korken (Naturkorken) erhalten wird. Andererseits ist es bekannt, aus zermahlenem Korkmaterial unter Verwendung von Bindemitteln einzeln oder im Stangenextrusionsverfahren sogenannte Agglomeratkorken zu formen oder Naturkorkenscheiben und Agglomeratkörper zu kombinierten Korken zusammenzufügen.

Zur Aufbereitung werden die geernteten und abgelagerten Platten aus Korkrinde (Korkholz) durch einen Kochprozeß, bei dem durch die Veränderung der Zellstruktur die hohe Elastizität des Korks erreicht wird, für die weitere Verarbeitung vorbereitet. Dabei können aufgrund von verunreinigtem Wasser Kontaminanten in das Korkmaterial eindringen. Bei der nachfolgenden Lagerung (Stabilisierung) über einen Zeitraum von in der Regel bis zu 21 Tagen wird üblicherweise durch Trocknen an der Luft der Wassergehalt der Korkplatten auf einen gewünschten Wert eingestellt. In dieser Zeit können sich Keime oder Pilze im und auf dem Korkmaterial entwickeln.

Aus der Korkrinde werden anschließend die Naturkorken mit entsprechenden Vorrichtungen ausgestanzt und einer chemischen Bleichung unterzogen, die vorzugsweise auf Peroxidbasis oder auch durch Chlorbleiche erfolgt. Danach erfolgt die Weiterbearbeitung mit unterschiedlichen Außenbehandlungsmitteln. Naturkorkscheiben für Sektkorken oder für aus Naturkorkscheiben und Agglomeratkörpern kombinierte Weinkorken werden aus dünnen Lagen von Naturkork ausgestanzt, von denen zuvor die Außenschichten entfernt wurden.

Agglomeratkorken werden aus den vermahlenen, nicht genutzten Resten der Naturkorkenproduktion (ca. 60% einer Korkplatte) hergestellt. Das Granulat wird je nach der zu produzierenden Qualität der Agglomeratkorken mehr oder weniger stark gereinigt, mit Bindemitteln (z.B. Polyurethan-Kleber) vermischt und im Strangverfahren oder im Einzelstückverfahren geformt. Im Falle kombinierter Korken werden Agglomeratkörper und Naturkorkscheiben mit Hilfe unterschiedlicher Kleber verbunden.

Im Verlauf dieser unterschiedlichen Produktionsprozesse kann es.aus verschiedenen Gründen zu einer starken Zunahme der Mikroorganismen auf und im Kork bzw. Korken kommen, sei es durch Neukontamination von außen oder durch eine Verbesserung ihrer Lebensbedingungen aufgrund der Erhöhung von Temperatur und Feuchtigkeit im Inneren. Die derzeitig eingesetzten Verklebetechniken erfordern lange Stabilisierungsphasen, in deren Verlauf es zu einer starken Zunahme der Mikroorganismenzahl kommen kann.

Parallel dazu kann auf sehr unterschiedlichen Wegen ein Eintrag chemischer Kontaminanten in das Korkmaterial erfolgen. Das Zusammenwirken von mikrobieller Stoffwechselaktivität mit der Präsenz chemischer Kontaminanten kann dann zur Bildung intensiv riechender Stoffwechselprodukte führen, wie zum bekannten 2,4,6-Trichloranisol, dem Verursacher des typischen Korkgeschmacks. Dessen Bildung geht nach heutiger Kenntnis allein auf eine mikrobielle Metabolisierung chemischer Vorstufen zurück. Zwei andere Stoffwechselprodukte sind das 1-Octen-3-ol, Verursacher des intensiven Geruches nach Waldpilzen, und das Guaiacol, Verursacher eines medizinischen/verbrannten Fehltones. Daneben können zahlreiche chemische Kontaminanten direkt, d.h. ohne eine zwischengeschaltete Metabolisierung, zu Fehltönen führen.

Zur Lösung oder Verringerung der benannten Probleme ist es in der korkenherstellenden Industrie bekannt, das Korkmaterial bzw. die Korken mit Peroxiden zu behandeln. Dies führt jedoch zu keiner tiefwirkenden Sterilisierung, da die Lösungen aufgrund der Undurchlässigkeit des Korks für Flüssigkeiten nicht oder nur in sehr geringem Maße eindringen. Darüber hinaus wird dabei eine Reduzierung der chemischen Kontaminanten nicht erreicht. Die Justierung des Wassergehaltes durch die derzeit eingesetzte Heißluftbehandlung ist nur mit großen Schwankungen möglich, da es sich beim Kork um einen außerordentlich guten Wärmeisolator handelt.

Aus der AU-A-1 855 583 ist ein Verfahren zur Behandlung von Flaschenverschlüssen aus Kork bekannt, wobei die Korken zur Sterilisierung zunächst einem Oxidationsmittel-Bad ausgesetzt und anschließend in einem Neutralisationsmittel-Bad behandelt werden, das das Oxidationsmittel neutralisieren soll. Anschließend können die Korken einer Gamma- oder Mikrowellenbestrahlung ausgesetzt werden. Dieses Verfahren ist aufwendig und seine Wirksamkeit ist von dem schwierig zu beherrschenden Zusammenspiel des Oxidationsmittels und des Neutralisationsmittels abhängig.

Aus der IT-B-1 268 708 ist es bekannt, Korkprodukte durch Bestrahlung mit Mikrowelle zu sterilisieren. Zu diesem Zweck werden die Korkprodukte auf einem Förderband durch eine Mikrowellenkammer befördert. Weitere Angaben insbesondere zu der Dauer und Intensität der Mikrowellenbehandlung sind in dieser Druckschrift nicht gemacht.

Der Erfindung liegt die Aufgabe zugrunde, ein Verfahren der genannten Art zu schaffen, mit dem nicht nur die Oberfläche, sondern auch das Innere des jeweiligen Korkens erreicht und die angestrebte Sterilisierung erzielt wird, ohne die Korksubstanz selbst zu schädigen, und das unter wirtschaftlich interessanten Bedingungen durchgeführt werden kann, wobei zusätzlich Kontaminationen der Korken reduziert werden.

Diese Aufgabe wird durch die Verfahren mit den in den unabhängigen Ansprüchen wiedergegebenen Merkmalen gelöst und durch die Merkmale der Unteransprüche gefördert.

Um eine gleichmäßige Behandlung zu gewährleisten, werden - je nach der Produktionsphase, in der die Mikrowelle zum Einsatz kommt-Korkholzplatten (z.B. im Anschluß an den Kochvorgang) oder bereits ausgestanzte Korken, Korkscheiben, Korkgranulat, frisch verklebte Korken oder andere Zwischen- und Endprodukte in einer entsprechenden Schüttung auf Förderbändern durch eine Mikrowellenanlage transportiert und mit Mikrowellen bestrahlt.

Da die zu behandelnden Materialien von unterschiedlicher Zusammensetzung, Qualität und Wassergehalt sind, müssen die eingesetzten Energien sowie die Einwirkzeiten der Mirkrowellen auf die jeweiligen Anfangsbedingungen (Kork- bzw. Korkentyp, Korkenqualität, Wassergehalt) und die gewünschten Endergebnisses abgestimmt sein.

Energien und Einwirkzeiten werden daher für den Bedarfsfall festgelegt und durch geeignete analytische Methoden überprüft. Sie hängen von weiteren Randbedingungen ab, wie der Feuchtigkeit und der Temperatur der Umgebungsluft, sowie der Größe und der Konfiguration der verwendeten Mikrowellenanlage.

Um eine gleichmäßige Durchstrahlung der zu behandelnden Korken sicherzustellen, werden die Korken entweder in einem geschlossenen Mikrowellenofen oder vorzugsweise in einem kontinuierlichen Mikrowellen-Banddurchlauf-Ofen bestrahlt. Eine Bestrahlungsdosis von 15 bis 1000 W pro kg Korken während einer Bestrahlungszeit von 2,5 bis 30 Minuten hat sich als geeignet erwiesen. Normalerweise wird mit einer Mikrowellenleistung des Ofens von 0,1 bis 4 kW/h pro kg Kork, vorzugsweise ca. 0,5 kW/h pro kg, eine gute bis sehr gute Sterilität des Korkmaterials erreicht. Darunter liegende Leistungen führen lediglich zu einer partiellen Austrocknung des Korks, aber nicht zu einer Sterilisierung. Im Gegenteil werden bei niederen Bestrahlungsdosen erhöhte Kontaminationen gegenüber dem nicht mikrowellenbehandelten Kork beobachtet, was darauf zurückgeführt wird, daß im Korkinneren Temperaturen von 30 bis 40°C erzeugt werden, welche für eine verstärkte Vermehrung der Mikroorganismen günstig sind. Eine über die angegebene Leistung hinausgehende Bestrahlung führt zu einer Überhitzung des Korkgefüges und damit zu einer zu starken Austrocknung und Zersetzung, so daß solche Behandlungen zu vermeiden sind. In der Regel wird eine vollständige Sterilität erreicht. Damit wird das Risiko der mikrobiellen Bildung sensorisch wirksamer chemischer Verbindungen im Korken, die als Hauptursache späterer negativer Aroma- und Geschmacksveränderungen in Wein, Sekt (Schaumweine) und anderen mit Korken verschlossenen Getränken angesehen wird, deutlich.vermindert.

Als Nebeneffekt der Sterilisation hat sich herausgestellt, daß von Haus aus noch relativ stark durchfeuchtete Korken mit einem Wassergehalt von etwa 6 bis 20%, wie sie aus der Bleiche und anschließenden Waschstationen erhalten werden, durchgehend auf eine gleichmäßige Feuchtigkeit von etwa 4 bis 10% getrocknet werden, wie sie für eine Verarbeitung optimal ist.

Auch die Reduzierung chemischer Kontaminanten wird unter diesen Bedienungen befriedigend erreicht. Das Risiko einer direkten Fehltonbildung durch Migration von Kontaminanten, die ohne den Zwischenschritt einer mikrobiellen Metabolisierung zu Aroma- und Geschmacksveränderungen führen können, wird durch die Behandlung deutlich vermindert, ebenso wie das Risiko einer Fehltonbildung durch Migration von Kontaminanten, die erst nach mikrobieller Metabolsierung zu sensorisch aktiven Verbindungen umgewandelt werden.

Bei der Herstellung von Agglomeratkorken bzw. von kombinierten Korken, die aus Agglomeratkorken und Naturkorkenscheiben (z.B. Sektkorken, sog. 1+1 Korken) bestehen, führt das Verfahren zu einer schnelleren Polymerisation und Aushärtung der derzeit verwendeten Polyurethanbindemittel. Eine besondere Verbesserung wird bei dem derzeitig verwendeten "Scheibenkleber" erreicht (Verbindung zwischen dem Agglomeratrohling des Sektkorkens bzw. des 1+1 Korkens und den Naturkorkscheiben), bei dem die sogenannte "Stabilisierungsphase" nach dem Verkleben und vor der weiteren Verarbeitung deutlich verkürzt werden kann.

Durch die weitaus präzisere Justierung des Wassergehaltes des Korks und der Forkenzwischenprodukte mit Hilfe des neuen Verfahrens, wird eine deutlich höhere Qualität bei der Applikation und Haftung der Außenbehandlungsmittel erreicht, die das Abdichtverhalten des Korkens bestimmen und darüber hinaus ein problemloses Ver- und Entkorken der Flaschen gewährleisten.

Der entscheidende Vorteil der Mikrowellenbehandlung gegenüber thermischen Verfahren ist in einem Grundproblem aller Arten thermischer Behandlung von Kork begründet: seine überaus geringe Wärmeleitfähigkeit. Durch eine thermische Behandlung, die von außen einwirkt, bleibt die erforderliche Temperaturerhöhung auf die Außenbereiche des Korks bzw. der Korken beschränkt und nimmt zum Inneren des Korken hin kontinuierlich ab.

Die Mikrowelle hingegen erregt mit Lichtgeschwindigkeit und dabei mit sehr großen Eindringtiefen das Wasser und andere durch Mikrowelle erregbare Moleküle, die sich im behandelten Material befinden, und führt zu einer sehr schnellen und sehr gleichmäßigen Durchwärmung bzw. Erhitzung des behandelten Materials ohne die Ausbildung von Temperaturgradienten. Je höher der Wassergehalt des zu behandelnden Materials, desto effektiver ist die Transformation der Mikrowelle in Wärme. Darin begründet sich die keimreduzierende bzw. sterilisierende Wirkung der Mikrowelle, die auf einer sehr effektiven Transformation der Mikrowelle in Wärme im Zellwasser der Mikroorgansimen beruht, die daraufhin abgetötet werden.

Unter Produktionsbedingungen kann die Entwicklung des Wassergehaltes des Mikrowelle-behandelten Materials mittels entsprechender Sensortechnik kontinuierlich überwacht werden (kontaktlose IR-Messtechnik, Pyrometer etc.), da mit abnehmendem Wasseranteil und damit geringer werdender Umwandlung der Mikrowellenenergie in Wärmeenergie ein genau vorherberechenbarer Temperaturverlauf im behandelten Material auftritt.

Die.Reduktion chemischer Kontaminationen beruht entweder:
- auf einer direkten Erregbarkeit der zu entfernenden Verbindungen und damit zu einer Erwärmung und einem steigenden Dampfdruck der jeweiligen Komponente, was zu einem schnellen Ausgasen des Korkmaterials führt,
   und/oder
- auf co-destillativen Effekten, die bei der Verdampfung von Wasser aus dem behandelten Korkmaterial auftreten,
   und/oder
- auf einer verstärkten Ausgasung aufgrund der durch die Anregung des Wassers steigenden Temperatur im Korkmaterial.

In einer bevorzugten Ausführungsform wird das Korkmaterial zu den vorstehend genannten Zwecken kontinuierlich durch einen Mikrowellen-Banddurchlauf-Ofen gefördert. Selbstverständlich ist es auch möglich, andere bekannte Typen von Mikrowellen-Durchlauferhitzern oder Mikrowellen-Durchlauftrocknern, wie sie seit einigen Jahren auf dem Markt verfügbar sind, einzusetzen.

Der versuchsweise eingesetzte Mikrowellen-Durchlauf-Ofen erlaubt es, die Mikrowellenleistung über eine Heizzone von ca. 2000 mm bis auf 4,0 kW einzustellen, wobei das zu bestrahlende Gut auf einem Transportband mit Bandgeschwindigkeiten zwischen 0 und 2 m/min bei einer Transportbandbreite von 290 mm gefördert werden kann. Zur Untersuchung der Sterilisierung von Korken wurden diese nebeneinander auf das Transportband aufgelegt und mit Durchlaufgeschwindigkeiten von 2 bis 10 Minuten durch den Ofen gefördert. Die Mikrowellenleistung wurde dabei zwischen 500 und 4000 Watt eingestellt. Wie der Tabelle 1 zu entnehmen ist, wird die mikrobielle Aktivität eines unbehandelten Korkens durch schwache Mikrowellenbestrahlung gesteigert, wobei eine bis auf das Doppelte gesteigerte Aktivität auftreten kann, um anschließend bei noch höherer Bestrahlungsdichte, wie sie erfindungsgemäß anzuwenden ist, schlagartig auf näherungsweise Null absinken.

**Tabelle 1: die Mikrowellenbehandlung von Korken zur Sterilisation:**

| Energieaufnahme pro kg Kork in Watt | mikrobielle Aktivität (CORA Faktor) |
|---|---|
| unbehandelt | 1,075 |
| 1,5 | 1,055 |
| 10 | 1,260 |
| 15 | 2,020 |
| 100 | 0,019 |

Tabelle 2 zeigt die Reduktion von drei beispielhaften chemischen Kontaminationen (Trichlormethan, Dichlorbenzol und 2,4,6-Trichloranisol). Die Mikrowellenbehandlung wurde dabei mit einer Bestrahlung von 4 kW bei einer Ofendurchlaufzeit von 10 Minuten und einer Korkmenge von 1 kg entsprechend der für die erfolgreiche Sterilisierung sinnvollen Bestrahlung durchgeführt. Es zeigt sich, daß von beiden Produkten etwa 90% ausgetrieben werden. Die Ausgasung dürfte dabei sowohl auf einer Erhöhung des Dampfdrucks der jeweiligen Komponenten durch die Erwärmung des Korkmaterials als auch auf einer co-destillativen Verdampfung zusammen mit dem ausgetretenen Wasser beruhen.

**Tabelle 2: die Mikrowellenbehandlung von Korken zur chemischen Dekontamination:**

| | | |
|---|---|---|
| a) | | |
| | Energieaufnahme pro kg Kork in Watt | Trichlormethan µg/g Kork |
| | | |
| | unbehandelt | 125 |
| | 100 | 12,5 |
| b) | | |
| | Energieaufnahme pro kg Kork in Watt | Dichlorbenzol µg/g Kork |
| | | |
| | unbehandelt | 8,9 |
| | 100 | 0,89 |
| c) | | |
| | Energieaufnahme pro kg Kork in Watt | 2,4,6-Trichloranisol ng/g Kork |
| | | |
| | unbehandelt | 25 |
| | 100 | 2,5 |

Im folgenden sollen die Produktionsverfahren für die unterschiedlichen Korkenarten näher erläutert werden:

In allen Fällen wird die Rinde der Korkeiche zunächst geerntet, anschließend einem Alterungsprozeß unterzogen und gekocht. Zur Stabilisierung und Vorab-Sterilisierung bzw. -Dekontamination werden die Korkrindenstücke dann mit Mikrowellenenergie von 15 bis 250 W pro kg Kork beaufschlagt, wobei die aufgebrachte Energie von der Feuchtigkeit der Korkrindenstücke abhängig ist.

Bei der Herstellung von einstückigen Naturkorken werden diese anschließend aus den Korkrindenstücken ausgestanzt, woraufhin sie einer erneuten Bestrahlung mit Mikrowellen mit einer Leistung von 50 bis 200 W pro kg Kork ausgesetzt werden, wodurch eine weitgehende Sterilisierung und Dekontamination erreicht wird. In gleichartiger Weise werden auch die für Sekt- und Champagnerkorken verwendeten Naturkorkscheiben hergestellt und behandelt.

Nach dem Schleifen der-Naturkorken werden diese mit H₂O₂ (Peroxid) gebleicht und anschließend mittels erneuter Bestrahlung mit Mikrowellenenergie von 15 bis 150 W pro kg Kork auf einen gewünschten Feuchtigkeitsgehalt getrocknet.

Bei der Herstellung von Agglomeratkorken bzw. kombinierten Korken wird aus Materialresten, die bei dem Ausstanzen der Naturkorken entstehen, ein Granulat hergestellt, das hinsichtlich seiner Feuchtigkeit mittels Bestrahlung mit Mikrowellenenergie von 50 bis 200 W pro kg Kork auf einen gewünschten Wert eingestellt und dabei gleichzeitig sterilisiert und dekontaminiert wird. Anschließend wird aus dem Granulat unter Hinzufügung eines Klebers in Einzelanfertigung oder im Strangpreßverfahren ein Korkenrohling erzeugt, der mit zuvor hergestellten Naturkorkscheiben zusammengeführt und mit diesen verklebt wird. Zur Stabilisierung der Verklebung werden die so hergestellten Korken einer Bestrahlung mit Mikrowellenenergie von 20 bis 150 W pro kg Kork ausgesetzt, wobei gleichzeitig eine annähernd vollständige Sterilisierung und Dekontamination erreicht wird.

Zwischen der Herstellung von Korken und ihrer Auslieferung an den Kunden können manchmal vier bis sechs Wochen vergehen, so daß es im Einzelfall passieren kann, daß die Korken eine zu geringe Feuchtigkeit von < 4% aufweisen. In diesem Fall werden die Korken durch Aufsprühen von Wasser befeuchtet und anschließend wird der Feuchtigkeitsgehalt durch Einwirkung von Mikrowellenenergie auf ein gewünschtes Maß eingestellt, wobei nochmals eine Sterilisierung und Dekontamination erreicht wird.

## Patentansprüche

1. Verfahren zur Herstellung von einstückigen Naturkorken, wobei die Rinde der Korkeiche zunächst geerntet, anschließend einem Alterungsprozess unterzogen und gekocht wird, wobei die Korkrindenstücke zur Stabilisierung und Vorab-Sterilisierung bzw. -Dekontamination dann mit Mikrowellenenergie von 15 bis 250 W pro kg Kork beaufschlagt werden und wobei die.Naturkorken aus den Korkrindenstücken ausgestanzt und anschließend zur Dekontamination und Sterilisation einer Bestrahlung mit Mikrowellen mit einer Leistung von 50 bis 200 W pro kg Kork ausgesetzt werden, dann geschliffen und mit H₂O₂ (Peroxid) gebleicht werden und anschließend mittels einer erneuten Bestrahlung mit Mikrowellen mit einer Leistung von 15 bis 150 W pro kg Kork getrocknet werden.

2. Verfahren nach Anspruch 1, **dadurch gekennzeichnet, daß** die Naturkorken in einem kontinuierlich arbeitenden Mikrowellen-Banddurchlaufofen bestrahlt werden.

3. Verfahren nach Anspruch 1, **dadurch gekennzeichnet, daß** die Naturkorken, die eine Feuchtigkeit von < 4% aufweisen, 4 bis 6 Wochen nach der Herstellung durch Aufsprühen von Wasser befeuchtet werden und anschließend der Feuchtigkeitsgehalt durch Einwirken von Mikrowellenenergie auf ein gewünschtes Maß eingestellt wird.

4. Verfahren nach Anspruch 1, **dadurch gekennzeichnet, daß** die Naturkorken von der Behandlung einen Wassergehalt von 6 bis 20 Gew.% und nach der Bestrahlung einen Wassergehalt von 4 bis 10 Gew.% aufweisen.

5. Verfahren zur Herstellung von kombinierten Korken, wobei die Rinde der Korkeiche zunächst geerntet, anschließend einem Alterungsprozess unterzogen und gekocht wird und die Korkrindenstücke zur Stabilisierung und Vorab-Sterilisation bzw. -Dekonatmination dann mit Mikrowellenenergie von 15 bis 250 W pro kg Kork beaufschlagt werden und wobei die Naturkorkscheiben aus den Korkrindenstücken ausgestanzt und anschließend zur Dekontamination und Sterilisation einer Bestrahlung mit Mikrowellen mit einer Leistung von 50 bis 200 W pro kg Kork ausgesetzt werden und wobei aus Korkresten ein Granulat hergestellt wird, das hinsichtlich seiner Feuchtigkeit mittels Bestrahlung mit Mikrowellenenergie von 50 bis 200 W pro kg Kork auf einen gewünschten Wert eingestellt und dabei gleichzeitig sterilisiert und dekontaminiert wird, wobei anschließend aus dem Granulat unter Hinzufügung eines Klebers in Einzelanfertigung oder im Stanzpressverfahren ein Korkenrohling erzeugt wird, der mit den zuvor hergestellten Naturkorkscheiben zusammengeführt und mit diesen verklebt wird.

6. Verfahren nach Anspruch 5, **dadurch gekennzeichnet, daß** die verklebten Korken zur Stabilisierung der Verklebung einer Bestrahlung mit Mikrowellen mit einer Leistung von 20 bis 150 W pro kg Kork ausgesetzt werden.

## Claims

1. Method for manufacturing one-piece natural corks, the cork oak bark initially being harvested, subjected to an ageing process and boiled, the cork bark pieces then being subject to a microwave energy of 15 to 250 W/kg of cork for stabilization and presterilization/predecontamination and the natural corks are punched out of the cork bark pieces and then, for decontamination and sterilization, irradiated with microwaves having a power of 50 to 200 W/kg of cork, ground and bleaches with H₂O₂ (peroxide) and then dried by further irradiation with microwaves having a power of 15 to 150 W/kg of cork.

2. Method according to claim 1, **characterized in that** the natural corks are irradiated in a continuously operating microwave continuous strip oven.

3. Method according to claim 1, **characterized in that** the natural corks having a moisture level of < 4%, are moistened 4 to 6 weeks after manufacture by spraying on water and then the moisture content is set to a desired level through the action of microwave energy.

4. Method according to claim 1, **characterized in that**, prior to treatment, the natural corks have a water content of 6 to 20 wt.% and after irradiation a water content of 4 to 10 wt.%.

5. Method for manufacturing combined corks, the cork oak bark firstly being harvested, followed by undergoing an ageing process and boiling and, for stabilization and presterilization/predecontamination, the cork bark pieces are then exposed to microwave energy of 15 to 250 W/kg of cork and the natural cork disks are punched out of the cork bark pieces and then, for decontamination and sterilization, exposed to irradiation with microwaves having a power of 50 to 200 W/kg of cork and from the cork residues is produced a granulate which, with respect to its moisture level, is set to a desired value by irradiation with microwave energy of 50 to 200 W/kg of cork and simultaneously sterilized and decontaminated, in which subsequently from the granulate and accompanied by the addition of an adhesive, in a single piece production or in a blanking out process a cork blank is produced, which is brought together with the previously manufactured natural cork disks and bonded therewith.

6. Method according to claim 5, **characterized in that** the bonded corks for stabilizing the bond are exposed to irradiation with microwaves having a power of 20 to 150 W/kg of cork.

## Revendications

1. Procédé de fabrication de bouchons en liège naturel d'une seule pièce, selon lequel l'écorce du chêne-liège est d'abord récoltée, ensuite soumise à un processus de vieillissement et cuite, procédé selon lequel les morceaux d'écorce de liège sont alors soumis à une énergie de micro-ondes de 15 à 250 W par kilo de liège pour les stabiliser et les préstériliser ou prédécontaminer, et selon lequel les bouchons en liège naturel sont découpés à la matrice dans les morceaux d'écorce de liège et ensuite soumis à une irradiation par micro-ondes d'une puissance de 50 à 200 W par kilo de liège pour les décontaminer et les stériliser, puis poncés et blanchis au peroxyde (H₂O₂) et ensuite séchés par une nouvelle irradiation par micro-ondes d'une puissance de 15 à 150 W par kilo de liège.

2. Procédé selon la revendication 1, **caractérisé en ce que** les bouchons en liège naturel sont irradiés en continu dans un four à micro-ondes à bande.

3. Procédé selon la revendication 1, **caractérisé en ce que** les bouchons en liège naturel, qui présentent une humidité inférieure à 4%, sont humidifiés par aspersion d'eau 4 à 6 semaines après leur fabrication et **en ce que** l'on amène ensuite la teneur en humidité à un degré souhaité sous l'effet d'une énergie à micro-ondes.

4. Procédé selon la revendication 1, **caractérisé en ce qu'**avant traitement, les bouchons en liège naturel présentent une teneur en humidité de 6 à 20 % du poids et après irradiation une teneur en humidité de 4 à 10 % du poids.

5. Procédé de fabrication de bouchons combinés, selon lequel l'écorce du chêne-liège est d'abord récoltée, ensuite soumise à un processus de vieillissement et cuite, procédé selon lequel les morceaux d'écorce de liège sont alors soumis à une énergie de micro-ondes de 15 à 250 W par kilo de liège pour les stabiliser et les préstériliser ou prédécontaminer, et selon lequel les disques de liège naturel sont découpés à la matrice dans les morceaux d'écorce de liège et ensuite soumis à une irradiation par micro-ondes d'une puissance de 50 à 200 W par kilo de liège pour les décontaminer et les stériliser, et selon lequel on fabrique un granulat à partir des restes de liège, dont on amène l'humidité à une valeur souhaitée au moyen d'une irradiation par énergie de micro-ondes d'une puissance de 50 à 200 W par kilo de liège et qui est ainsi simultanément stérilisé et décontaminé, selon lequel on produit ensuite à partir du granulat, en y ajoutant une colle, soit par fabrication à l'unité, soit par estampage, une ébauche de bouchon, qui est assemblée et collée aux disques de liège naturel précédemment produits.

6. Procédé selon la revendication 5, **caractérisé en ce que** les bouchons collés sont soumis à une irradiation par micro-ondes d'une puissance de 20 à 150 W par kilo de liège pour stabiliser le collage.
